# EUROPÄISCHE PATENTSCHRIFT

(11) **EP 1 104 683 B1**
(45) Veröffentlichungstag und Bekanntmachung des Hinweises auf die Patenterteilung: **20.04.2005**
(21) Anmeldenummer: 99123948.4
(22) Anmeldetag: 03.12.1999
(51) Int. Cl.: A61M 5/46, A61D 7/00, A61K 9/00

(54) **Kombination aus einer Vorrichtung zur Injektion von Antiparasitika bei Haustieren und einem antiparasitischen Wirkstoff**
Combination comprising a device for the injection of antiparasitic agents into pet animals and an antiparasitic agent
Combination du dispositif d'injection d'agents antiparasitaires pour des animaux de compagnie et d'agent antiparasitaire

(43) Veröffentlichungstag der Anmeldung: 06.06.2001
(73) Patentinhaber: Mattern, Udo, 6370 Stans (CH)
(72) Erfinder: Mattern, Claudia Dr., 6370 Stans (CH)
(74) Vertreter: Winkler, Andreas, Dr.

(56) Entgegenhaltungen:
- EP-A- 0 692 270
- EP-A- 0 836 851
- WO-A-94/13342
- WO-A-94/23777
- WO-A-96/21427
- McEWAN D. et al: 'Route of passage of cypermethrin across the surface of sheep skin', RESEARCH IN VETERINARY SCIENCE, 1986, No.41, Seiten 237-241
- COCHET P. et al: 'Skin distribution of fipronil by microautoradiography following topical administration to the beagle dog', EUROPEAN JOURNAL OF DRUG METABOLISM AND PHARMACOKINETICS, 1997, Vol. 22, No.3, Seiten 211-216
- STENDEL W. et al: 'Analytical determination of the distribution of flumethrin on the body surface of cattle following topical pour-on application', VETERINARY PARASITOLOGY, 1992, No. 42, Seiten 127-143

## Beschreibung

Die Erfindung betrifft eine Kombination aus einer Vorrichtung zur Injektion einer Wirkstofformulierung bei Haustieren und einer Wirkstofformulierung mit einem antiparasitisch wirksamen Wirkstoff in einer zur Injektion geeigneten Formulierung.

Die EP 0 897 728 A1 offenbart eine Injektionsvorrichtung nach Art eines Stiftes, mit der eine einstellbare Menge einer Flüssigkeit, die in dem Stift in einem Vorratsbehälter vorliegt, durch Bewegen eines Druckknopfes in Längsrichtung des Stiftes aus dem Vorratsbehälter entnommen und durch eine Kanüle in ein Lebewesen injiziert werden kann. Die Offenbarung befaßt sich insbesondere mit einer sicheren Halterung des Vorratsbehälters, z. B. einer Arzneimittelphiole, und dem Aufbau der einstellbaren Dosierungsvorrichtung.

US-Patent 5,964,731 offenbart eine Halterung für eine Lanzette oder eine Injektionsnadel, die es ermöglicht, das zum Durchstechen geeignete Teil aus einer Hülse hervortreten zu lassen und nach Gebrauch wieder in die Hülse zurückzuziehen, wobei überdies das zum Stechen geeignete Teil in der zurückgezogenen Stellung von einem Deckel, der innerhalb der Hülse angeordnet ist, abgedeckt wird. Insbesondere sieht diese Offenbarung vor, die Abdeckung, die sich nach Gebrauch über die in die Hülse zurückgezogene Nadel oder Lanzette stülpt, so auszugestalten, daß dieser Vorgang irreversibel ist und die Vorrichtung kein zweites Mal verwendet werden kann.

Die WO 99/27984 offenbart eine Injektionsvorrichtung zum medizinischen Gebrauch, mit der insbesondere pastöse Fluide mittels eines Elektroantriebes durch einen Kolben aus dem Vorratszylinder gepreßt werden und einer Injektionsnadel zugeführt werden können.

EP 0 836 851 A1 betrifft antiparasitisch wirksame Amidin-Verbindungen und deren Applikationsformen.

Bekannte Injektionsvorrichtungen eignen sich zur Injektion flüssiger oder auch pastöser Arzneimittelformulierungen, wobei die Dosis einstellbar ist oder durch den Aufbau festgelegt sein kann. Die jeweils offenbarten Längen der Injektionsnadeln eignen sich zur intramuskulären oder intravenösen Injektion. Im Unterschied dazu sieht die vorliegende Erfindung eine gezielte Injektion der Wirkstofformulierung in bestimmte Hautschichten direkt unterhalb der Hautoberfläche eines Tieres vor.

Antiparasitische Wirkstoffe werden bei Tieren entweder oral bzw. intramuskulär verabreicht oder aber äußerlich auf die Haut aufgetragen. Äußerlich aufgetragene antiparasitische Wirkstoffe können durch die Hautschichten des Tieres diffundieren und sich anschließend systemisch im Tier verteilen, wenn sie ein relativ niedriges Molekulargewicht aufweisen, ausreichend lipophil sind und in einer Formulierung mit permeationsverstärkenden Stoffen, wie beispielsweise 2-Dimethylsulfoxid, 2-Pyrrolidon, Ethanol, Harnstoff etc., als Sorptionsvermittler aufgebracht werden.

Nachteilig bei der äußerlichen Anwendung ist, daß der antiparasitische Wirkstoff mit einem großen Anteil Sorptionsvermittler korremuliert werden muß, um die Diffusionsbarriere der Haut für den Wirkstoff durchgängig zu machen und dadurch die natürliche Hautfunktion gestört wird. Weiterhin muß bei der äußerlichen Anwendung die Formulierung im Überschuß eingesetzt werden, da nur ein Anteil des aufgebrachten antiparasistischen Wirkstoffes tatsächlich in die Haut diffundiert und der verbleibende Anteil an die Umgebung als Schadstoff abgegeben wird, ohne beim Tier wirksam werden zu können. Die wesentlichen Einflußfaktoren, die den in die Hautschicht eindiffundierenden Anteil der aufgetragenen antiparasitischen Formulierung beeinflussen, umfassen den spezifischen Zustand des behandelten Hautgebietes, d. h. die besondere Beschaffenheit einer bestimmten Körperzone, Hautalter und - feuchtigkeit sowie die individuell unterschiedlichen Eigenschaften der Haut. Daher ist es schwierig, die zur Erzielung der angemessenen aufgenommenen Dosis des antiparasitischen Wirkstoffes erforderliche Menge der antiparasitischen Formulierung für ein bestimmtes Tier abzuschätzen.

Der vorliegenden Erfindung stellt sich vor diesem Stand der Technik die Aufgabe, eine gattungsgemäße Kombination bereitzustellen, mit der ein antiparasitischer Wirkstoff sicher und zuverlässig einem Tier verabreicht werden kann.

Die erfindungsgemäße Lösung liegt darin, eine Kombination einer Injektionsvorrichtung mit einem antiparasitischen Wirkstoff, der in eine zur Injektion geigneten Formulierung eingearbeitet ist, bereitzustellen. Besonders bevorzugt ist hierbei, daß die Injektionsvorrichtung vom Stifttyp ist, bei der sich die abgegebene Menge der Wirkstofformulierung dosieren läßt. Weiterhin ist es für die vorliegende Erfindung wesentlich, daß die Eindringtiefe der in der Injektionsvorrichtung vorhandenen Injektionsnadel so bemessen ist, daß die Wirkstofformulierung nur direkt unter die obersten Hautschichten gespritzt wird und in die tieferen Hautregionen gelangt, jedoch nicht in das subkutane Fett- oder Muskelgewebe gespritzt wird. Als mögliche Ausführungsform eignen sich daher Injektionsvorrichtungen vom Stifttyp, bei denen die zur Verfügung stehende Länge der Injektionsnadel außerhalb des Stiftgehäuses nur um 3 mm, bevorzugt 2 mm, besonders bevorzugt 1 mm, hervorragt, so daß die Eindringtiefe der Injektionsnadel durch die Vorrichtung fest vorgegeben ist.

Die zur Injektion geeignete Formulierung des antiparasitischen Wirkstoffes enthält vorzugsweise nur einen geringen Anteil an Hilfsstoffen, wie beispielsweise Resorptionsvermittlern, so daß schon eine geringe Menge der antiparasitischen Formulierung ausreicht, um eine ausreichende Dosis des antiparasitischen Wirkstoffes zu verabreichen. Bevorzugt ist der antiparasitische Wirkstoff in einem kleinen Volumen der Formulierung enthalten. Besonders bevorzugt sind Formulierungen, bei denen eine ausreichende Dosis des antiparasitischen Wirkstoffes, die pro Kilogramm Gewicht des Tieres erforderlich ist, in einem Volumen von 100 µl, bevorzugt 50 µl, besonders bevorzugt 10 µl vorliegen.

Die Dosierung der antiparasitischen Formulierung kann mit der erfindungsgemäßen Vorrichtungskombination entweder so vorgenommen werden, daß die mit einem Injektionsstoß verabreichte Menge auf das Gewicht des Tieres einstellbar ist, beispielsweise durch eine Dosiervorrichtung gemäß der EP 0 897 728, oder daß ein baulich festgelegtes Dosiervolumen der Injektionsvorrichtung so bemessen ist, daß es für 1, 2, bevorzugt 5 kg Gewicht des Tieres bemessen ist und durch mehrmaliges Injizieren des gleichen Formulierungsvolumens die Dosis der antiparasitischen Formulierung bestimmt werden kann.

Ein Vorteil der erfindungsgemäßen Kombination aus einer Injektionsvorrichtung und einem antiparasitischen Wirkstoff in geeigneter Formulierung ist zum einen die genaue und reproduzierbare Dosierung der Formulierung, da Verluste durch frühzeitiges Abdampfen oder ungenügende Sorption durch die Haut praktisch nicht auftreten. Zum anderen ist es möglich, die erfindungsgemäße Kombination auch durch den Nichtmediziner gefahrlos zu verwenden, da die Injektionsvorrichtung nur eine geringe Eindringtiefe der Injektionsnadel erlaubt, so daß nur ein sehr geringes Verletzungsrisiko für das Tier besteht.

Besonders vorteilhaft ist, daß die vorliegende Erfindung auf die Verwendung großer Anteile an Sorptionsvermittlern in der Wirkstofformulierung verzichtet und daher die unerwünschten Wirkungen dieser Formulierungshilfsstoffe vermeidet.

Weiterhin ist es mit der vorliegenden Erfindung möglich, solche speziellen Formulierungen in die Hautschichten des Tieres zu injizieren, die als Depot für den antiparasitischen Wirkstoff fungieren.

## Patentansprüche

1. Kombination aus einer Vorrichtung zur Injektion einer Wirkstofformulierung bei Haustieren und einer Wirkstofformulierung mit einem antiparasitisch wirksamen Wirkstoff in einer zur Injektion geeigneten Formulierung, wobei die Vorrich-tung eine Injektionsvorrichtung ist, die so ausgelegt ist, daß die Injektionsnadel die obersten, aber nicht die unteren Hautschichten durchdringt, und nicht in das subkutane Fett- oder Muskel gewebe eindringt und die injizierbare Wirkstofformulierung als antiparasitischen Wirkstoff IGR-Chitinsynthesehemmer, Phosphorsäureester, Karbamate, Pyrethrum und Pyrethroide, Avermectine, Benzimidazole, Phenothiazine oder Praziquantel enthält.

2. Vorrichtung nach Anspruch 1, **dadurch gekennzeichnet, daß** die Eindringtiefe der Injektionsnadel maximal 3 mm beträgt.

3. Vorrichtung nach Anspruch 1, **dadurch gekennzeichnet, daß** die Eindringtiefe der Injektionsnadel maximal 2 mm beträgt.

4. Vorrichtung nach Anspruch 1, **dadurch gekennzeichnet, daß** die Eindringtiefe der Injektionsnadel maximal 1 mm beträgt.

5. Vorrichtung nach einem der vorangehenden Ansprüche, **dadurch gekennzeichnet, daß** die Injektionsvorrichtung so ausgelegt ist, daß ein festgelegtes Volumen der Wirkstofformulierung abgebbar ist.

6. Vorrichtung nach einem der Ansprüche 1 bis 4, **dadurch gekennzeichnet, daß** die Injektionsvorrichtung einen einstellbaren Dosiermechanismus aufweist.

## Claims

1. A combination consisting of a device for the injection of an active substance formulation into domestic animals and an active substance formulation having an antiparasitically active substance in a formulation suitable for injection, wherein the device is an injection device which is so designed that the injection needle penetrates the topmost but not the lower layers of the skin and does not enter the subcutaneous fat or muscle tissue and the injectable active substance formulation contains as antiparasitic active substance IGR chitin synthesis inhibitors, phosphoric acid esters, carbamates, pyrethrum and pyrethoids, avermectine, benzimidazoles, phenothiazines or praziquantel.

2. A device according to claim 1, **characterised in that** the depth of penetration of the injection needle is 3 mm maximum.

3. A device according to claim 1, **characterised in that** the depth of penetration of the injection needle is 2 mm maximum.

4. A device according to claim 1, **characterised in that** the depth of penetration of the injection needle is 1 mm maximum.

5. A device according to any of the preceding claims, **characterised in that** the injection device is so designed that a fixed volume of the active substance formulation can be dispensed.

6. A device according to any one of claims 1 to 4, **characterised in that** the injection device comprises an adjustable metering mechanism.

## Revendications

1. Combinaison d'un dispositif d'injection d'un principe actif chez des animaux de compagnie et d'un principe actif avec un agent antiparasitaire dans une formulation appropriée pour l'injection, dans laquelle le dispositif est un dispositif d'injection qui est prévu de sorte que l'aiguille d'injection pénètre dans les couches les plus supérieures de la peau mais pas dans les couches inférieures ni dans les tissus adipeux ou les tissus musculaires sous-cutanés et dans laquelle le principe actif injectable contient comme agent antiparasitaire un inhibiteur de synthèse de chitine-régulateur de croissance d'insectes, de l'ester phosphorique, du carbamate, du pyrèthre et du pyrèthroïde, de l'avermectine, du benzimidazole, de la phénothiazine ou du praziquantel.

2. Dispositif selon la revendication 1, **caractérisé en ce que** la profondeur de pénétration de l'aiguille d'injection est de 3 mm au maximum.

3. Dispositif selon la revendication 1, **caractérisé en ce que** la profondeur de pénétration de l'aiguille d'injection est de 2 mm au maximum.

4. Dispositif selon la revendication 1, **caractérisé en ce que** la profondeur de pénétration de l'aiguille d'injection est de 1 mm au maximum.

5. Dispositif selon l'une quelconque des revendications précédentes, **caractérisé en ce que** le dispositif d'injection est prévu de sorte qu'un volume établi du principe actif puisse être distribué.

6. Dispositif selon l'une des revendications 1 à 4, **caractérisé en ce que** le dispositif d'injection comporte un mécanisme de dosage réglable.
